(19) Europäisches Patentamt
European Patent Office
Office européen des brevets

(11) Publication number: **0 268 982 B1**

(12) **EUROPEAN PATENT SPECIFICATION**

(45) Date of publication of patent specification: **25.03.92**

(51) Int. Cl.⁵: **A61K 7/06**, A61K 7/08, A61K 7/48, C08G 77/06

(21) Application number: **87116974.4**

(22) Date of filing: **17.11.87**

(54) **Cosmetic compositions containing microemulsions of dimethylpolysiloxane.**

(30) Priority: **18.11.86 JP 274799/86**

(43) Date of publication of application:
**01.06.88 Bulletin 88/22**

(45) Publication of the grant of the patent:
**25.03.92 Bulletin 92/13**

(84) Designated Contracting States:
**DE FR GB**

(56) References cited:
**EP-A- 0 169 386**
**GB-A- 2 018 271**
**GB-A- 2 064 363**

**CHEMICAL ABSTRACTS, vol. 66, 1967, page 2806, abstract no. 29171e, Columbus, Ohio, US; D.R. WEYENBERG et al.: "Anionic emulsion polymerization of siloxanes", & AMER. CHEM. SOC., DIV. POLYMER CHEM., PREPRINTS 7(2), 562-8(1966)**

(73) Proprietor: **Toray Silicone Company, Ltd.**
**8, 2-chome Muro-machi Nihonbashi**
**Chuo-ku Tokyo 103(JP)**

(72) Inventor: **Harashima, Asao**
**6, 1-chome, Yshudai Nishi**
**Ichihara-shi Chiba Prefecture(JP)**
Inventor: **Tanaka, Osamu**
**6, 1-chome, Yushudai Nishi**
**Ichihara-shi Chiba Prefecture(JP)**
Inventor: **Maruyama, Tsuneo**
**6, 1-chome, Yushudai Nishi**
**Ichihara-shi Chiba Prefecture(JP)**
Inventor: **Ohta, Yayoi**
**423-20, Nitona-cho**
**Chiba-shi Chiba Prefecture(JP)**

(74) Representative: **Spott, Gottfried, Dr. et al**
**Patentanwälte Spott und Puschmann**
**Sendlinger-Tor-Platz 11**
**W-8000 München 2(DE)**

EP 0 268 982 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to cosmetic compositions based on dimethylpolysiloxane microemulsions. More specifically, this invention relates to cosmetic compositions which are based on dimethylpolysiloxane microemulsions produced by emulsion polymerization.

Dimethylpolysiloxanes have been used in various cosmetics, such as skin and hair cosmetics. For example, dimethylpolysiloxanes are important cosmetic components as they form uniform coatings on the surface of skin or hair, thereby providing smoothness, moisturization, and water repellency. However, dimethylpolysiloxanes are poorly miscible with the other components blended in cosmetics, such as, for example, alcohols, mineral oil, beeswax and fatty acid esters. A prior remedy to the miscibility problem has been to reduce the particle size of the emulsion through the use of particular surfactants in order to form a stable dispersion of the components.

Japanese Patent Application Laid Open Number 60-126209 (126,209/85) discloses an emulsified cosmetic which has an average particle size of approximately 2 micrometers and which is emulsified using a polyoxyalkylene group-containing organopolysiloxane. Japanese Patent Application Laid Open Number 60-197610 (197,610/85) discloses an emulsified cosmetic which has an average particle size of 0.2 to 2 micrometers and which is emulsified using a polyoxyalkylene group-containing organopolysiloxane, a surfactant with HLB $\geqq 10$, a linear saturated higher alcohol having 12 through 22 carbon atoms, and aqueous ethanol.

However, even cosmetics obtained by emulsification using the above specific surfactants suffer from a lack of long term stability due to the organopolysiloxane emulsion having an average particle size above 0.2 micrometers. As a consequence, when blended with other cosmetic starting materials, the organopolysiloxane will separate out during long-term storage.

In U.S. Patent No. 2 891 920 a method is described wherein a polyorganosiloxane is emulsified in water by means of anionic or cationic surfactants and then polymerized with strong mineral acids or strong alkalic catalysts. It is not possible to obtain with this process an emulsion having a particle size of $\leqq 0.15$ micrometers. Only an emulsion having a higher particle size and being opaque or milky can be obtained.

The object of the present invention is to eliminate the aforesaid problem by providing a cosmetic which is based on an organopolysiloxane microemulsion prepared by emulsion polymerization, and which has excellent storage stability. The object of the invention is achieved by means of a cosmetic composition based on a dimethylpolysiloxane microemulsion, which is produced by the emulsion polymerization of dimethylpolysiloxane. The microemulsion of the invention has an average particle size $\leqq 0.15$ micrometers.

The organopolysiloxane microemulsion of the present invention is produced by the emulsion polymerization of organopolysiloxane having a low degree of polymerization. The average particle size of the emulsion after emulsion polymerization must be $\leqq 0.15$ micrometers and preferably is $\leqq 0.12$ micrometers. When the average emulsion particle size exceeds 0.15 micrometers, the stability of the blend with the other components of the cosmetic declines, and the external appearance of the cosmetic will be very negatively affected. Furthermore, the degree of polymerization (DP) of the dimethylpolysiloxane after emulsion polymerization is preferably in the range of from 3 to 5,000. The degree of polymerization is even more preferably in the range of from 10 to 3,000. The molecular terminal ends of said dimethylpolysiloxane may consist of hydroxy groups; alkoxy groups such as methoxy, ethoxy, and propoxy; or trimethylsiloxy groups.

The emulsion of the invention can be produced, for example, by means of an emulsion polymerization process in which a crude emulsion, consisting of dimethylpolysiloxane having a low degree of polymerization, a first surfactant, and water, is slowly dripped into an aqueous solution containing a catalytic quantity of a polymerization catalyst and a second surfactant which acts as an emulsifying agent.

Low-DP dimethylpolysiloxanes suitable as a starting material for the crude emulsion are typically exemplified by cyclic dimethylpolysiloxane having the formula:

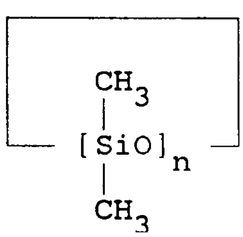

wherein n in the above formula is an integer having a value of from 3 to 10. The cyclic dimethylpolysiloxane

may be a single species, or a combination of 2 or more species. In addition to such dimethylpolysiloxane cyclics, it is also permissable to include small quantities of hydroxy-terminated dimethylpolysiloxane; silanes having hydrolyzable groups, such as dimethyldimethoxysilane, trimethylmethoxysilane, and dimethyldichlorosilane; hexamethyldisiloxane, for example.

A first surfactant is needed to convert the dimethylpolysiloxane into a crude emulsion. Anionic, cationic, and nonionic surfactants may be used to form the crude emulsion.

Anionic surfactants useful in the invention are exemplified by alkyl sulfates such as lauryl sulfate; alkylbenzenesulfonic acids such as hexylbenzenesulfonic acid, octylbenzenesulfonic acid, decylbenzenesulfonic acid, dodecylbenzenesulfonic acid, cetylbenzenesulfonic acid, and myristylbenzenesulfonic acid; the sulfate esters of monoalkyl polyoxyethylene ethers, for example of the formulas:

$CH_3-(CH_2)_6-CH_2O-(C_2H_4O)_2-SO_3H$;
$CH_3-(CH_2)_8-CH_2O-(C_2H_4O)_8-SO_3H$;
$CH_3-(CH_2)_{19}-CH_2O-(C_2H_4O)_4-SO_3H$; and
$CH_3-(CH_2)_8-CH_2C_6H_4O-(C_2H_4O)_2-SO_3H$;

and alkylnaphthylsulfonic acids.

Suitable cationic surfactants are exemplified by quaternary ammonium hydroxides such as tetramethylammonium hydroxide, octyltrimethylammonium hydroxide, dodecyl- trimethyl ammonium hydroxide, hexadecyltrimethylammonium hydroxide, octyldimethylbenzylammonium hydroxide, decyldimethylbenzylammonium hydroxide, didodecyldimethylammonium hydroxide, dioctadecyl dimethylammonium hydroxide, tallow trimethylammonium hydroxide, and cocotrimethylammonium hydroxide, as well as the corresponding salts of these materials.

Nonionic surfactants of the invention are exemplified by polyoxyalkylene alkyl ethers, polyoxyalkylene alkylphenol ethers, polyoxyalkylene alkyl esters, polyoxyalkylene sorbitan alkyl esters, polyethylene glycols, polypropylene glycols, and diethylene glycol.

The surfactant can be used in the form of a single type of surfactant, or the surfactant can be used as a combination of 2 or more types, with the exception that the combination of an anionic surfactant with a cationic surfactant is excluded. Thus, one can use a single type of anionic surfactant; a combination of 2 or more types of anionic surfactants; a single type of nonionic surfactant; the combination of 2 or more types of nonionic surfactants; a single type of cationic surfactant; the combination of 2 or more types of cationic surfactants; the combination of two or more types of surfactants selected from both the anionic and nonionic surfactants; and the combination of two or more types of surfactants selected from both the cationic and nonionic surfactants.

The surfactant is used in the crude emulsion in a quantity sufficient for the formation of an emulsion. This quantity will vary with the type of surfactant and therefore, cannot be specifically restricted. However, an amount of surfactant in the range of from 2 - 10 wt% is preferred. Water is used in the crude emulsion preferably in a quantity which gives a dimethylpolysiloxane concentration in the range of 10 - 40 wt%.

The crude emulsion is prepared by mixing the above described low-DP dimethylpolysiloxane, surfactant, and water to homogeneity. The mixture is then passed through an emulsifying device such as an homogenizer, colloid mill, or line mixer, for example.

The microemulsion of the present invention can be obtained by means of an emulsion polymerization process in which the crude emulsion is gradually dripped into a separately prepared aqueous solution which contains a catalytic quantity of a polymerization catalyst and the second surfactant.

The polymerization catalyst includes anionic and cationic catalysts. The anionic catalysts are exemplified by mineral acids such as hydrochloric acid, sulfuric acid, and phosphoric acid; by alkyl sulfates; and by alkylbenzenesulfonic acids, the sulfate esters of polyoxyethylene monoalkyl ethers, and alkylnaphthylsulfonic acids. Examples of some of the polymerization catalysts are given above in the description of the anionic surfactants of the invention. The cationic catalysts are exemplified by alkali metal hydroxides such as potassium hydroxide and sodium hydroxide, and by quaternary ammonium hydroxides and their salts. Examples of the latter cationic catalysts are given above in the description of the cationic surfactants of the invention.

The second surfactant used in polymerization can be the same as the first surfactant used for the crude emulsion. Accordingly, when an alkylbenzenesulfonic acid, sulfate ester of a polyoxyethylene monoalkyl ether, alkylnaphthylsulfonic acid, or quaternary ammonium hydroxide or salt thereof is used as the surfactant, it can also serve as the polymerization catalyst. Considering the ionicity of the emulsion, when an anionic surfactant is used in the crude emulsion, microemulsion production will then require the use of an anionic catalyst and an anionic and/or nonionic surfactant. When a cationic surfactant is used in the

crude emulsion, microemulsion production will require the use of a cationic catalyst and a cationic and/or nonionic surfactant. When a nonionic surfactant is used in the crude emulsion, production of the microemulsion will require the use of an anionic or cationic catalyst: an anionic and/or nonionic surfactant can be used with the anionic catalyst, while a cationic and/or nonionic surfactant can be used with a cationic catalyst.

The quantity of surfactant used in the catalyst plus the surfactant aqueous solution is to be 5 - 70 weight parts. Preferably this amount is in the range of from 25 - 60 weight parts per 100 weight parts dimethylpolysiloxane in the crude emulsion. At below 5 weight parts, the average particle size in the emulsion will not reach to ≦0.15 micrometers. The catalyst is used in amounts of 0.2 - 10 weight parts and preferably 0.5 - 5.0 weight parts per 100 weight parts dimethylpolysiloxane.

The aqueous catalyst solution is preferably at a temperature in the range of from 40 - 95 °C when the crude emulsion is dripped in. The dripping rate will vary depending on the concentration, type of catalyst and the temperature of the aqueous catalyst solution. Thus, at high catalyst concentrations, or at a high aqueous catalyst solution temperature, the crude emulsion can be added rapidly. However, a dropwise addition of 30 minutes or longer is preferred in order to produce microemulsions having smaller particle sizes. After the completion of addition, the microemulsion having an average particle size ≦0.15 micrometers is prepared by conducting emulsion polymerization at 0 - 90 °C to the desired viscosity. After emulsion polymerization, neutralization of the catalyst is preferably carried out using alkali in the case of an anionic polymerization catalyst and acid in the case of a cationic polymerization catalyst. Furthermore, while the dimethylpolysiloxane concentration during emulsion polymerization is not specifically restricted, it is preferably maintained in the range of from 5 - 50 wt%.

Cosmetics according to the invention can be obtained using the dimethylpolysiloxane microemulsion base as is, or by adding arbitrary quantities of freely selected components, as long as the object of the present invention is not adversely affected. The addition of 0 - 99.9 wt% of these freely selected components is preferred. These freely selected components are, for example, hydrocarbons such as liquid paraffin, Vaseline®, solid paraffins, squalane, olefin oligomers, for example, esters such as isopropyl palmitate, stearyl stearate, octyldodecyl myristate, octyldodecyl oleate, 2-ethylhexanoic acid triglyceride, etc.; higher alcohols such as lauryl alcohol, cetyl alcohol, stearyl alcohol, etc.; higher fatty acids such as palmitic acid, stearic acid, etc.; moisture retainers such as ethylene glycol, propylene glycol, 1,3-butylene glycol, glycerol, and sorbitol; inorganic powders such as titanium oxide, carbon black, iron oxide, sericite, talc, kaolin, and mica; organic powders such as nylon, polystyrene, polyethylene, and polyacrylate; solvents such as ethanol, etc.; water; various surfactants as exemplified for dimethylpolysiloxane emulsion polymerization; germicides; perfumes; etc.

The means by which the above freely selected components may be added to the cosmetic of the present invention include simply mixing the dimethylpolysiloxane microemulsion with these components to homogeneity. Alternatively, the freely selected components may be mixed to a homogeneous state using a stirrer, or may be emulsified in advance separately from the dimethylpolysiloxane microemulsion using an emulsifying device such as an homogenizer, colloid mill, line mixer, etc., followed by combination with the dimethylpolysiloxane microemulsion.

Embodiments of the invention include formulations for hair cosmetics such as a hair oil, hair dye, hair-dressing oil, setting lotion, stick pomade, curl-dressing oil, hair cream, hair tonic, hair liquid, hair spray, pomade, shampoo, hair rinse, hair conditioner, and hair lotion, for example. Other embodiments of the invention include hand cream, skin cream, foundation, eye shadow, and facial cleanser formulations. The cosmetic compositions can be formulated to have various physical forms, including solid, gel, liquid, and paste forms.

The invention will be explained in the following illustrative examples. In the examples, the term "part" is intended to mean weight part. The average particle size was measured using a Coulter Model N4 from Coulter Electronics, Inc. (USA).

EXAMPLE 1

560 Parts water, 30 parts dicocoyldimethylammonium chloride, and 400 parts cyclic dimethylsiloxane tetramer were placed in a 2 L beaker and mixed to a homogeneous state. This mixture was passed through an homogenizer emulsifier 3 times at 350 kg/cm$^2$ to produce a crude emulsion.

Separately, 870 parts water and 130 parts tallow trimethylammonium chloride were placed in a 5 L four-necked flask, followed by dissolving the tallow into an aqueous solution and letting the solution stand at a liquid temperature of 85 °C while being slowly stirred. Emulsion polymerization was conducted by slowly dripping the separately prepared crude emulsion into the aqueous tallow trimethylammonium chloride solution over 2 hours, followed by cooling, maintaining the temperature at 48 °C for 2 hours, and adjusting

the pH to 7.0 with phosphoric acid. A transparent microemulsion (emulsion A) was produced, which had an average particle size of 0.05 micrometers. Emulsion A was broken using methanol in order to extract the oil, whose structure was then identified by gel permeation chromatography using chromatograph equipment from Toyo Soda Kogyo Kabushiki Kaisha (Toyo Soda Mfg. Co., Ltd.) and infrared absorption spectral analysis using equipment from Kabushiki Kaisha Hitachi Seisaku-sho (Hitachi Ltd.). The results confirmed a trimethylsiloxy-terminated dimethylpolysiloxane having a degree of polymerization of 100.

Rinse 1 was then prepared by adding water to emulsion A to obtain a silicone concentrated of 0.4 wt%. Rinse 2 was prepared by diluting emulsion A using 5 wt% aqueous ethanol to obtain a silicone concentration of 0.4 wt%. Rinses 3 and 4, also having 0.4 wt% silicone concentrations, were prepared using 10.0 and 20.0 wt% aqueous ethanol, respectively.

Two 200 cc samples of each of rinses 1-4 were placed in glass bottles. One sample of each rinse was maintained at 25 ° C for 72 days to determine standing stability of the emulsion, while the other sample of each rinse was subjected to an environmental tester programed to execute a 0 to 50 ° C temperature cycle every 24 hours for 72 days to determine stability to temperature cycling. The stability of the samples was then observed by visual inspection.

COMPARATIVE EXAMPLE 1

80 Parts trimethylsiloxy-terminated dimethylpolysiloxane (viscosity = 350 centistokes), 8 parts polyoxyethylene lauryl ether (HLB = 11.5), and 12 parts water were mixed to a homogeneous state, followed by emulsification using a colloid mill and then dispersal to a homogeneous state in 300 parts water in order to prepare a mechanically formed emulsion (emulsion B). The emulsion thus formed had an average particle size of 0.8 micrometers. Rinses 5 through 8 having silicone concentrations of 0.4 wt% were prepared from emulsion B and, respectively, water, or 5, 10, or 20 wt% aqueous ethanol. As in Example 1, the standing stability and the temperature cycling stability were tested.

The results of the stability testing of the formulations of Example 1 and Comparative Example 1 are reported below in Tables 1 and 2.

Table 1

| rinse no. | number of days maintained at 25°C | | | | | |
|---|---|---|---|---|---|---|
| | 0 | 4 | 15 | 30 | 41 | 72 |
| Example 1 | | | | | | |
| 1 | transparent | transparent | transparent | transparent | transparent | transparent |
| 2 | transparent | transparent | transparent | transparent | transparent | transparent |
| 3 | transparent | transparent | transparent | transparent | transparent | transparent |
| 4 | transparent | transparent | transparent | transparent | transparent | transparent |
| Comp. Example 1 | | | | | | |
| 5 | turbid | turbid | turbid | turbid | turbid | turbid |
| 6 | turbid | turbid | turbid | turbid | separation | separation |
| 7 | turbid | turbid | turbid | separation | separation | separation |
| 8 | turbid | separation | separation | separation | separation | separation |

Table 2

| rinse no. | number of days subjected to 0 - 50°C cycling | | | | | |
|---|---|---|---|---|---|---|
| | 0 | 4 | 15 | 30 | 41 | 72 |
| Example 1 | | | | | | |
| 1 | transparent | transparent | transparent | transparent | transparent | transparent |
| 2 | transparent | transparent | transparent | transparent | transparent | transparent |
| 3 | transparent | transparent | transparent | transparent | transparent | transparent |
| 4 | transparent | transparent | transparent | transparent | transparent | transparent |
| Comp. Example 1 | | | | | | |
| 5 | turbid | turbid | turbid | turbid | turbid | separation |
| 6 | turbid | turbid | turbid | separation | separation | separation |
| 7 | turbid | turbid | separation | separation | separation | separation |
| 8 | separation | separation | separation | separation | separation | separation |

The increase in the combability of hair (expressed in %) after using rinses 1 and 4 of Example 1, and rinses 5 and 8 of Comparative Example 1 was evaluated as detailed below and reported in Table 3. The rinses were tested both immediately after their production and after being subjected to 0 to $50^{O}$ ° C temperature cycling for 72 days.

Hair treatment: A hair bundle (12 g, 20 cm length) was washed with a 1 wt% solution of aqueous sodium lauryl sulfate, rinsed with hot water, and then dried using a drier. The hair bundle was immersed in water for 30 seconds, wrapped in a towel, and then drained under a pressure of 20 kg/314 $cm^2$ for 5 seconds. After removal from the towel, the bundle was brushed until a smooth run was obtained.

Hair rinse treatment: The pretreated hair was immersed in a rinse for 1 minute, immersed in hot water for 30 seconds, wrapped in a towel, and then drained under a pressure of 20 kg/314 $cm^2$ for 5 seconds. After removal from the towel, the bundle was brushed until a smooth run was obtained.

Measurement of combability: A comb was installed in the mobile holder of a Universal Tensile Tester commercially available from Tester Sangyo Kabushiki Kaisha . One end of the treated hair was immobilized, and the bundle was then placed between the teeth of the comb of the mobile holder. The maximum value of the tensile force was read when the mobile holder was moved at a rate of 200 mm/minute.

This procedure was carried out both on hair which had received only the pretreatment, and hair which had been treated with the rinse, and the increase in combability was calculated using the following formula:

increase in combability (%) = [(A -B)/A] x 100

wherein A = tensile force for hair receiving only the pretreatment; and
B = tensile force for hair treated with rinse.

The results of the combability testing are set forth in Table 3 below.

Table 3

| rinse number | percent increase in combability | |
|---|---|---|
| | immediately after production | after standing 72 days subject to 0 - 50°C temp. cycling |
| Example 1 | | |
| 1 | 53 | 50 |
| 4 | 51 | 52 |
| Comparative Example 1 | | |
| 5 | 31 | 16 |
| 8 | 25 | 10 |

EXAMPLE 2

850 Parts water, 10 parts dodecylbenzensulfonic acid, and 600 parts cyclic dimethylsiloxane tetramer were placed in a 2 L beaker and stirred to a homogeneous state. This mixture was passed through an homogenizer emulsifier four times at 400 kg/cm$^2$ to form a crude emulsion.

Separately, 1,300 parts water and 180 parts dodecylbenzenesulfonic acid were placed in a 5 L four-neck flask, followed by dissolving into an aqueous solution and then standing at a liquid temperature of 85° C with gentle stirring. Emulsion polymerization was conducted by gradually adding the crude emulsion dropwise over two hours to the aqueous dodecylbenzenesulfonic acid solution. Following cooling, maintenance at 48° C for 2 hours, and adjusting the pH to 7.0 using 50 wt% aqueous sodium hydroxide emulsion C was formed which had an average particle size of 0.05 micrometers.

Emulsion C was broken using methanol to extract the oil. The structure of the oil, identified using gel permeation chromatography and infrared absorption spectroscopy, was found to be a hydroxy-terminated dimethylpolysiloxane having a degree of polymerization of 1,200.

Rinses 9 to 12, having silicone concentrations of 0.4 wt%, were prepared using emulsion C and water, or respectively, 5, 10, or 20% aqueous ethanol. The rinses were tested, as in Example 1, for standing stability at 25° C for 72 days and also tested for cyclic temperature stability by being subject to the 0 to 50° C temperature cycle for 72 days. These results are reported in Tables 4 and 5, respectively.

Also as in Example 1, the increase in hair combability of rinses 9 and 12 was tested, both immediately after preparation of the rinse and after being maintained in the environmental tester for 72 days. These results are reported in Table 6.

Table 4

| rinse no. | number of days maintained at 25 ° C | | | | | |
|---|---|---|---|---|---|---|
| | 0 | 4 | 15 | 30 | 41 | 72 |
| Example 2 | | | | | | |
| 9 | transparent | transparent | transparent | transparent | transparent | transparent |
| 10 | transparent | transparent | transparent | transparent | transparent | transparent |
| 11 | transparent | transparent | transparent | transparent | transparent | transparent |
| 12 | transparent | transparent | transparent | transparent | transparent | transparent |

Table 5

| rinse no. | number of days subjected to 0 - 50 ° C cycling | | | | | |
|---|---|---|---|---|---|---|
| | 0 | 4 | 15 | 30 | 41 | 72 |
| Example 2 | | | | | | |
| 9 | transparent | transparent | transparent | transparent | transparent | transparent |
| 10 | transparent | transparent | transparent | transparent | transparent | transparent |
| 11 | transparent | transparent | transparent | transparent | transparent | transparent |
| 12 | transparent | transparent | transparent | transparent | transparent | transparent |

Table 6

| rinse number | increase in combability (%) | |
|---|---|---|
| | immediately after preparation | after standing 72 days subject to 0 - 50 ° C temp. cycling |
| Example 2 | | |
| 9 | 47 | 45 |
| 12 | 46 | 46 |

EXAMPLE 3

Shampoo compositions 1 to 4, having the compositions set forth in Table 7, were prepared from emulsion A (as prepared in Example 1) and emulsion C (as prepared in Example 2). The compositions were subjected to temperature cycling as in Example I in order to investigate the stability of the materials after storage for 72 days.

Additionally, a foaming test of each shampoo composition was conducted. After being subjected to temperature cycling for 72 days, a sample of each shampoo composition was diluted 100-fold with water, and 10 mL of this dilution was placed in a 100 mL measurement cylinder. The cylinder was then vigorously shaken 10 times. The foam volume thereby produced was measured after 1 uminute. These results are reported in Table 7.

COMPARATIVE EXAMPLE 2

Shampoo compositions 5 and 6, as shown in Table 7, were prepared from emulsion B (as prepared in Comparative Example 1). Stability and foaming tests as in Example 3 were conducted on these compositions, and the results of the tests are reported in Table 7.

Table 7

| | Example 3 | | | | Comparative Example 2 | |
|---|---|---|---|---|---|---|
| | shampoo composition no. | | | | shampoo composition no. | |
| | 1 | 2 | 3 | 4 | 5 | 6 |
| composition (in parts) | | | | | | |
| sodium lauryl sulfate | 10 | 10 | — | 10 | 10 | — |
| coco fatty acid diethanolamide | 5 | 5 | — | 5 | 5 | — |
| lauryl sulfate triethanolamine salt | — | — | 15 | — | — | 15 |
| cationic polymer Merquat 550 * | 1 | — | — | — | — | — |
| emulsion A | 2 | 5 | — | — | — | — |
| emulsion B | — | — | — | — | 5 | 5 |
| emulsion C | — | — | 2 | 5 | — | — |
| water | 82 | 80 | 83 | 80 | 80 | 80 |
| evaluation | | | | | | |
| stability after temperature cycling for 72 days | transparent | transparent | transparent | transparent | separation | separation |
| foaming test (mL) | 10 | 10 | 9.5 | 11 | 3 | 2 |

* available from Merck & Company, Inc., Rahway, New Jersey

EXAMPLE 4

Hair conditioning compositions 1 to 4, having the compositions as given in Table 8, were prepared using emulsion A (as prepared in Example 1) or emulsion C (as prepared in Example 2). The stability of the hair conditioning compositions when maintained under temperature cycling was tested for 72 days, as in Example 1. Also, the increase in combability was tested by the method of Example 1, but using, in place of the rinses, hair conditioning compositions 1 to 4 which had been subjected to 72 days of temperature cycling and then diluted 2-fold with water. These results are reported in Table 8.

COMPARATIVE EXAMPLE 3

Hair conditioning composition 5, the composition of which is set forth in Table 8, was prepared from emulsion B (as prepared in Comparative Example 1). Hair conditioning composition 5 was then tested as in Example 4. These results are reported in Table 8.

Table 8

| | Example 4 | | | | Comparative Example 3 |
|---|---|---|---|---|---|
| | hair conditioning composition no. | | | | hair conditioning composition no. |
| | 1 | 2 | 3 | 4 | 5 |
| composition (in parts) | | | | | |
| stearyldimethyl-ammonium chloride | 1.0 | 1.0 | 1.0 | 1.0 | 1.0 |
| cetanol | 1.0 | 1.0 | 1.0 | 1.0 | 1.0 |
| stearyl alcohol | 1.0 | 1.0 | 1.0 | 1.0 | 1.0 |
| liquid paraffin | 2.0 | 2.0 | 2.0 | 2.0 | 2.0 |
| polyoxyethylene (DP = 6) stearyl ether | 1.0 | 1.0 | 1.0 | 1.0 | 1.0 |
| propylene glycol | 5.0 | 5.0 | 5.0 | 5.0 | 5.0 |
| emulsion A | 2.0 | 5.0 | — | — | — |
| emulsion B | — | — | — | — | 5.0 |
| emulsion C | — | — | 2.0 | 5.0 | — |
| water | 87 | 84 | 87 | 84 | 84 |
| evaluation | | | | | |
| stability after standing under temperature cycling for 72 days | trans-parent | trans-parent | trans-parent | trans-parent | separa-tion |
| increase in combability (%) | 65 | 67 | 60 | 62 | 50 |

EXAMPLE 5

Jelly-like hair treatment compositions 1 to 3, having the compositions given in Table 9, were prepared and then tested as in Example 1 for stability when subjected to temperature cycling for 72 days. The increase in combability was tested as in Example 1, in place of the rinses, hair treatment compositions 1 to

3 were used directly after maintenance under temperature cycling for 72 days. Furthermore, after testing the increase in combability, the treated hair was dried, and the luster of the hair was visually evaluated. The results are reported in Table 9.

COMPARATIVE EXAMPLE 4

Hair treatment composition 4, as set forth in Table 9, was prepared using emulsion B (as prepared in Comparative Example 1). The composition was tested as in Example 5, and the results of the testing are reported in Table 9.

Table 9

| | Example 5 | | | Comparative Example 4 |
|---|---|---|---|---|
| | hair treatment composition no. | | | hair conditioning comp. no. |
| | 1 | 2 | 3 | 4 |
| composition (in parts) | | | | |
| carboxymethyl-cellulose | 0.5 | 0.5 | 0.5 | 0.5 |
| glycerol | 34.2 | 34.2 | 34.2 | 34.2 |
| triethanolamine | 2.3 | 2.3 | 2.3 | 2.3 |
| emulsion A | 2.0 | — | — | — |
| emulsion B | — | — | — | 2.0 |
| emulsion C | — | 2.0 | 5.0 | — |
| evaluation | | | | |
| stability after standing under temperature cycling for 72 days | transparent | transparent | transparent | separation |
| increase in combability (%) | 50 | 52 | 59 | 30 |
| luster | strongly lustrous | strongly lustrous | strongly lustrous | moderately lustrous |

EXAMPLE 6

The hair dye compositions in Table 10 were prepared as two part solutions. The first parts, or first solutions for hair dye compositions 1 and 2 were prepared with the compositions as shown in Table 10. The stability of the first solutions of the hair dye compositions was tested by subjecting them to temperature cycling as in Example 1 to determine the status of the materials after storage for 72 days. After temperature cycling testing the first solutions of hair dye compositions, the respective second solutions were combined with the first solutions in order to prepare hair dye liquids. Hair bundles (length = 12 cm, weight = 12 g) were dyed by immersion in 500 mL of the particular hair dye for 20 minutes. The hair bundles were then immersed in and washed with 1 wt% aqueous sodium lauryl sulfate for 10 minutes, and were then thoroughly rinsed in warm water at 40 ° C. The hair bundles were then dried in a convection oven at 50 ° C for 1 hour. In the blank test, hair dying was similarly carried out, but was dyed using a first solution of the hair dye composition from which emulsion A had been omitted. The dyed hair was evaluated by ten judges, and Table 10 reports the number of judges who rendered an evaluation that the hair exhibited a deeper black color than that of the blank.

COMPARATIVE EXAMPLE 5

First and second solutions of a hair dye composition 3, having the composition given in Table 10, were prepared. This composition was tested as in Example 6, and the results are reported in Table 10.

Table 10

| | Example 6 | | Comparative Example 5 | |
|---|---|---|---|---|
| | hair dyeing composition no. | | hair dye composition no. | blank |
| | 1 | 2 | 3 | |
| composition (in parts) | | | | |
| first solution | | | | |
| p-phenylenediamine | 2 | 2 | 2 | 2 |
| resorcinol | 0.1 | 0.1 | 0.1 | 0.1 |
| oleic acid | 20 | 20 | 20 | 20 |
| aqueous ammonia (28%) | 10 | 10 | 10 | 10 |
| polyoxyethylene (DP = 6) stearyl ether | 15 | 15 | 15 | 15 |
| emulsion A | 5 | — | — | — |
| emulsion B | — | — | 5 | — |
| emulsion C | — | 5 | — | — |
| water | 50 | 50 | 50 | 50 |
| second solution | | | | |
| 5 wt% aqueous hydrogen peroxide | 6 | 6 | 6 | 6 |
| water | 94 | 94 | 94 | 94 |
| evaluation | | | | |
| stability after standing under temperature cycling for 72 days | stable | stable | separation | — |
| number of judges scoring a deeper black (in ten judges) | 10 | 10 | 2 | — |

EXAMPLE 7

Cold permanent compositions 1 and 2, having the compositions given in Table 11, were prepared. The stability of the first solutions of these cold permanent compositions was tested as in Example 1 by subjecting the first solutions to temperature cycling for 72 days.

Furthermore, 12 g hair bundles (length = 20 cm) wrapped on curlers (diameter = 25 mm, width = 70

mm) were respectively immersed for 10 minutes at 25 °C in 1,000 mL of each of cold permanent compositions 1 and 2 obtained from the 72 day temperature cycling stability test. Each hair bundle was then removed from the curler, washed in 1,000 mL 1 wt% aqueous sodium lauryl sulfate, and thoroughly rinsed with hot water.

The combability of the hair bundles was measured by the method described in Example 1. The luster of the hair bundles was evaluated visually after drying at room temperature. After the evaluation of luster, cellotape was wound around the hair bundle, and it was suspended in an environmental tester at 30 °C/90% RH for 24 hours in order to evaluate curl retention. Curl retention is expressed as:

$$\frac{\text{wave length before standing}}{\text{wave length after standing for 24 hours}}$$

These results are reported in Table 11.

COMPARATIVE EXAMPLE 6

Cold permanent compositions 3 and 4, having the compositions given in Table 11, were prepared. Testing was conducted as in Example 7 on these compositions, and the results are reported in Table 11.

Table 11

| | Example 7 | | Comparative Example 6 | |
|---|---|---|---|---|
| | cold perm composition no. | | cold perm composition no. | |
| | 1 | 2 | 3 | 4 |
| composition (in parts) | | | | |
| first solution | | | | |
| 5 wt% aqueous thioglycolic acid | 50 | 50 | 50 | 50 |
| 5 wt% aqueous ammonia | 50 | 50 | 50 | 50 |
| polyoxyethylene (DP = 12) lauryl ether | 1.0 | 1.0 | 1.0 | 1.0 |
| stearyltrimethylammonium chloride | 0.2 | 0.2 | 0.2 | 0.2 |
| sodium edetate | 0.2 | 0.2 | 0.2 | 0.2 |
| emulsion A | 5.0 | 5.0 | — | — |
| emulsion B | — | — | 5.0 | — |
| emulsion C | — | 5.0 | — | — |
| second solution | | | | |
| 5 wt% aqueous potassium bromate | 100 | 100 | 100 | 100 |
| evaluation | | | | |
| luster | strongly lustrous | strongly lustrous | lustrous, but not strongly so | absent |
| curl retention (mm): before standing/after standing for 24 hours | 100/120 | 110/128 | 118/149 | 111/161 |
| increase in combability (%) | 36 | 35 | 18 | 0 |
| stability after standing under temperature cycling for 72 days | trans-parent | trans-parent | not trans-parent | trans-parent |

EXAMPLE 8

Skin cream compositions 1 and 2, having the compositions given in Table 12, were prepared as follows. The aqueous-phase components were separately heated to 70 °C and then mixed to a homogeneous state. The same procedure was followed for the oil-phase components. The oil-phase liquid mixture and aqueous-phase liquid mixture were combined, mixed further with a homomixer, and then cooled. When the

temperature reached 45°C, the silicone component was added, and skin cream compositions 1 and 2 were obtained by further cooling to room temperature (25°C) while mixing to a homogeneous state.

Using these compositions, temperature-cycling stability was tested as in Example 1 over a period of 72 days. The results of this testing are reported in Table 12.

COMPARATIVE EXAMPLE 7

Skin cream compositions 3, having the composition given in Table 12, was prepared according to the procedure described in Example 8. This composition was subjected to the same testing as in Example 1, and the results of the testing are reported in Table 12.

Table 12

| | Example 8 | | Comparative Example 7 |
|---|---|---|---|
| | skin cream composition no. | | skin cream composition no. |
| | 1 | 2 | 3 |
| composition (in parts) | | | |
| oil-phase components | | | |
| beeswax | 1.5 | 1.5 | 1.5 |
| cetanol | 5.5 | 5.5 | 5.5 |
| stearic acid | 8.0 | 8.0 | 8.0 |
| squalane | 10.0 | 10.0 | 10.0 |
| propylene glycol monostearate | 3.0 | 3.0 | 3.0 |
| polyoxyethylene stearyl ether | 1.0 | 1.0 | 1.0 |
| perfume | trace | trace | trace |
| aqueous-phase components | | | |
| methylparaben | 0.1 | 0.1 | 0.1 |
| glycerol | 12.0 | 12.0 | 12.0 |
| triethanolamine | 1.0 | 1.0 | 1.0 |
| water | 60 | 60 | 60 |
| silicone components | | | |
| emulsion A | 1.0 | — | — |
| emulsion B | — | — | 1.0 |
| emulsion C | — | 1.0 | — |
| evaluation | | | |
| stability after standing under temperature cycling for 72 days | the entire surface of the cream has a uniform luster, and no substances have separated | the entire surface of the cream has a uniform luster, and no substances have separated | the surface of the cream has a pearly luster with silicone separation |

EXAMPLE 9

Liquid foundation compositions 1 and 2, as given in Table 13, were prepared according to the procedure as follows:

1. Preparation of the pigment components: the specified pigment components were thoroughly mixed and ground in a ball mill.
2. Preparation of the aqueous-phase components: the water was heated to 70 ° C, the bentonite was added, and this was thoroughly mixed by stirring. The propylene glycol and glycerol were then both dispersed in the sodium carboxycellulose, and this was added to and dissolved in the aqueous bentonite, followed by addition and dissolving of the triethanolamine and methylparaben.
3. Preparation of the oil-phase components: All the oil-phase components were heated and melted at 80 ° C.
4. Addition of pigments to aqueous phase: The pigment components, prepared as in procedure step 1, were added to the aqueous-phase components, followed by dispersion to a homogeneous state using a homomixer and maintenance at 70 ° C.
5. Addition of the oil-phase: The oil-phase components held at 80 ° C, prepared as in procedure step 3, were mixed into the aqueous pigment dispersion held at 70 ° C which had been prepared in procedure step 4. After cooling to 50 ° C, the silicone component was added, followed by thorough mixing until the temperature reached 25 ° C to obtain the liquid foundation compositions.

The temperature cycling stability of the liquid foundation compositions was tested as in Example 1 over a 72 day period. The results of this testing are reported in Table 13.

COMPARATIVE EXAMPLE 8

A liquid foundation composition 3, having the composition given in Table 13, was prepared following the procedures of Example 9, and was then similarly tested. The results of the testing are reported in Table 13.

Table 13

| | Example 9 | | Comparative Example 8 |
|---|---|---|---|
| | liquid foundation composition no. | | liquid foundation composition no. |
| | 1 | 2 | 3 |
| composition (in parts) | | | |
| oil-phase components | | | |
| stearic acid | 2.4 | 2.4 | 2.4 |
| isostearyl alcohol | 1.0 | 1.0 | 1.0 |
| vaseline | 2.0 | 2.0 | 2.0 |
| squalane | 3.0 | 3.0 | 3.0 |
| 2-ethylhexanoic acid triglyceride | 10.0 | 10.0 | 10.0 |
| propylparaben | 0.05 | 0.05 | 0.05 |
| aqueous-phase components | | | |
| water | 65 | 65 | 65 |
| sodium carboxycellulose | 0.2 | 0.2 | 0.2 |
| bentonite | 0.5 | 0.5 | 0.5 |
| glycerol | 2.0 | 2.0 | 2.0 |
| propylene glycol | 2.0 | 2.0 | 2.0 |
| triethanolamine | 1.1 | 1.1 | 1.1 |
| methylparaben | 0.1 | 0.1 | 0.1 |
| pigment components | | | |
| titanium oxide | 8.0 | 8.0 | 8.0 |
| talc | 4.0 | 4.0 | 4.0 |
| red iron oxide | 0.5 | 0.5 | 0.5 |
| silicone components | | | |
| emulsion A | 2.0 | — | — |
| emulsion B | — | — | 1.0 |
| emulsion C | — | 2.0 | — |
| evaluation | | | |
| surface status after standing under temperature cycling for 72 days | surface is uniform and smooth | surface is uniform and smooth | minute oil droplets observed, surface slightly nonuniform |

Because the cosmetic of the present invention is based on a dimethylpolysiloxane microemulsion which has an average particle size ≧0.15 micrometers and which is prepared by the emulsion polymerization of dimethylpolysiloxane, it characteristically has excellent stability when blended with other cosmetic starting materials and additionally has excellent storage stability, making it quite useful in the art.

## Claims

1. A cosmetic composition comprising a dimethylpolysiloxane microemulsion, wherein said microemulsion is formed by the emulsion polymerization of dimethylpolysiloxane, the average particle size of said microemulsion being ≦ 0.15 micrometers.

2. A composition comprising a dimethylpolysiloxane microemulsion, wherein said microemulsion is formed by the emulsion polymerization of dimethylpolysiloxane of the general formula:

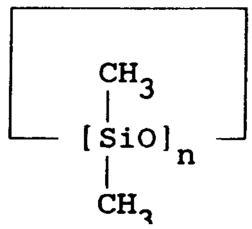

wherein n is an integer having a value of from 3 to 10, theaverage particle size of said microemulsion being ≦ 0.15 micrometers and the degree of polymerization of the microemulsion polymerized dimethylpolysiloxane is from 3 - 5,000.

3. A composition having an emulsion particle size of ≦ 0.15 micrometers formed by the process comprising:

forming a crude emulsion from water, an amount of a first surfactant (A) sufficient to form said crude emulsion and dimethylpolysiloxane of the general formula:

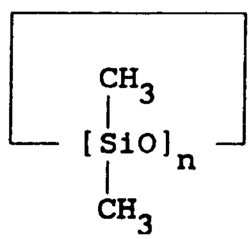

wherein n is an integer having a value of from 3 - 10;

gradually adding said crude emulsion to an aqueous solution containing a catalytic quantity of an emulsion polymerization promoting catalyst and a second surfactant (B);

carrying out emulsion polymerization at a temperature range of from 0 - 90 °C to reach a predetermined viscosity;

then neutralizing said polymerization catalyst.

4. A composition as claimed in Claim 3, wherein said anionic surfactants of groups (A) and (B) are selected from the group consisting of alkyl sulfates, alkylbenzenesulfonic acids the sulfate esters of monoalkyl polyoxyethylene ethers, and alkylnaphthyl-sulfonic acids,

said cationic surfactants of groups (A) and (B) are selected from the group consisting of quaternary ammonium hydroxides, and the corresponding salts of these materials, and

said nonionic surfactants of groups (A) and (B) are selected from the group consisting of polyoxyalkylene alkyl ethers, polyoxyalkylene alkylphenol ethers, polyoxyalkylene alkyl esters, polyoxyalkylene sorbitan alkyl esters, polyethylene glycols, polypropylene glycols, and diethylene glycol.

5. A composition as claimed in claim 3, wherein said first surfactant is contained in said crude emulsion in a amount of from 2 - 10 wt.%, and water is contained in said crude emulsion in an amount of from 10 - 40 wt%, the balance of said crude emulsion being dimethylpolysiloxane.

6. A composition as claimed in claim 3, wherein said second surfactant is contained in said aqueous solution in an amount of from 5 - 70 weight parts per 100 weight parts of said dimethylpolysiloxane in said crude emulsion.

7. A cosmetic composition comprising a microemulsion having an emulsion particle size of ≦ 0.15 micrometers formed by the process comprising:

making a crude emulsion from water, an amount of a first surfactant sufficient to form said crude emulsion and dimethylpolysiloxane of the general formula:

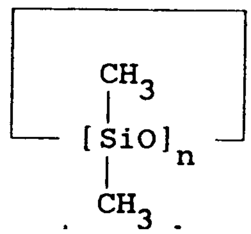

wherein n is an integer having a value of from 3 - 10; gradually adding said crude emulsion to an aqueous solution containing a catalytic quantity of an emulsion polymerization promoting catalyst and a second surfactant; carrying out emulsion polymerization at a temperature range of from 0 - 90 ° C to reach a predetermined viscosity; then neutralizing said polymerization catalyst; wherein said first surfactant is selected from the group (A) consisting of cationic, anionic and nonionic surfactants, and said first surfactant is a single surfactant or combination of surfactants selected from said group (A), with the exception that a cationic surfactant cannot be combined with an anionic surfactant, said second surfactant is selected from the group (B) consisting of cationic, anionic and nonionic surfactants, and said second surfactant is a single surfactant or combination of surfactants selected from said group (B), with the exception that a cationic surfactant cannot be combined with an anionic surfactant; and said polymerization catalyst is selected from the group consisting of cationic and anionic polymerization catalysts, and quaternary ammonium hydroxides, with the requirements:

when said first surfactant is anionic said polymerization catalyst must be anionic and said second surfactant is selected from said group (B) anionic and nonionic surfactants;

when said first surfactant is cationic said polymerization catalyst must be cationic and said second surfactant is selected from said group (B) cationic and nonionic surfactants;

when said first surfactant is nonionic and said polymerization catalyst is anionic said second surfactant is selected from said group (B) anionic and nonionic surfactants; and

when said first surfactant is nonionic and said polymerization catalyst is cationic said second surfactant is selected from said group (B) cationic and nonionic surfactants,

and from 0 - 99.9 wt% of freely selected cosmetic components based on the total amount of the cosmetic composition selected from the group consisting of hydrocarbons, esters, higher alcohols, higher fatty acids, moisture retainers, inorganic powders, organic powders, solvents such as ethanol, surfactants, water, germicides and perfumes.

8. A cosmetic composition as claimed in claim 7, wherein said hydrocarbons are selected from the group consisting of liquid paraffin, petroleum jelly, solid paraffins, and squalane,

said olefin oligomers are selected from the group of esters consisting of isopropyl palmitate, stearyl stearate, octyldodecyl myristate, octyldodecyl oleate, and 2-ethylhexanoic acid triglyceride,

said higher alcohols are selected from the group consisting of lauryl alcohol, cetyl alcohol, and stearyl alcohol,

said higher fatty acids are selected from the group consisting of palmitic acid and stearic acid,

said moisture retainers are selected from the group consisting of ethylene glycol, propylene glycol, 1,3-butylene glycol, glycerol, and sorbitol,

said inorganic powders are selected from the group consisting of titanium oxide, carbon black, iron oxide, sericite, talc, kaolin, and mica,

said organic powders are selected from the group consisting of nylon, polystyrene, polyethylene, and polyacrylate, and

said catalysts/surfactants are selected from the group consisting of hydrochloric acid, sulfuric acid, phosphoric acid, potassium hydroxide, sodium hydroxide, tetramethylammonium hydroxide, octyltrimethylammonium hydroxide, dodecyltrimethyl ammonium hydroxide, hexadecyltrimethylammonium hydroxide, octyldimethyl benzylammonium hydroxide, decyldimethylbenzylammonium hydroxide, didodecyldimethylammonium hydroxide, dioctadecyl dimethylammonium hydroxide, tallow trimethylammonium hydroxide, and cocotrimethylammonium hydroxide.

## Revendications

1. Une composition cosmétique comprenant une microémulsion de diméthylpolysiloxane, dans laquelle ladite microémulsion est formée par polymérisation en émulsion de diméthylpolysiloxane, la dimension particulaire moyenne de ladite microémulsion étant inférieure ou égale à 0,15 micromètre.

2. Une composition comprenant une microémulsion de diméthylpolysiloxane, dans laquelle ladite microémulsion est formée par polymérisation en émulsion de diméthylpolysiloxane répondant à la formule générale

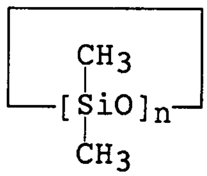

dans laquelle n est un entier ayant une valeur de 3 à 10, la dimension particulaire moyenne de ladite microémulsion étant inférieure ou égale à 0,15 micromètre et le degré de polymérisation du diméthylpolysiloxane polymérisé en microémulsion étant de 3 à 5000.

3. Une composition ayant une dimension particulaire d'émulsion inférieure ou égale à 0,15 micromètre formée par le procédé comprenant :

   la formation d'une émulsion brute à partir d'eau, d'une quantité d'un premier agent tensioactif (A) suffisante pour former ladite émulsion brute et de diméthylpolysiloxane de formule générale :

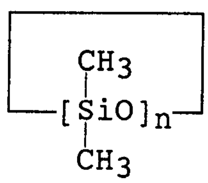

   dans laquelle n est un entier ayant une valeur de 3 à 10 ;

   l'addition graduelle de ladite émulsion brute à une solution aqueuse contenant une quantité catalytique d'un catalyseur de polymérisation en émulsion et un deuxième agent tensioactif (B) ;

   la polymérisation en émulsion dans un domaine de températures de 0 à 90 ° C pour atteindre une viscosité prédéterminée ;

   puis la neutralisation dudit catalyseur de polymérisation.

4. Une composition selon la revendication 3, dans laquelle lesdits agents tensioactifs anioniques des groupes (A) et (B) sont choisis dans le groupe formé par les sulfates d'alkyle, les acides alkylbenzènesulfoniques, les esters sulfates d'éthers monoalkyliques de polyoxyéthylène et les acides alkylnaphtylsulfoniques,

   lesdits agents tensioactifs cationiques des groupes (A) et (B) sont choisis dans le groupe formé par les hydroxydes d'ammonium quaternaire et les sels correspondants de ces corps, et

   lesdits agents tensioactifs non ioniques des groupes (A) et (B) sont choisis dans le groupe formé par les éthers de polyoxyalkylène et d'alkyle, les éthers de polyoxyalkylène et d'alkylphénol, les esters alkyliques de polyoxyalkylène, les esters alkyliques de polyoxyalkylène sorbitane, les polyéthylène glycols, les polypropylène glycols et le diéthylène glycol.

5. Une composition selon la revendication 3, dans laquelle ledit premier agent tensioactif est présent dans ladite émulsion brute en une proportion de 2 à 10 % en poids, et de l'eau est présente dans ladite émulsion brute en une proportion de 10 à 40 % en poids, le complément à 100 % de ladite émulsion brute étant du diméthylpolysiloxane.

**6.** Une composition selon la revendication 3, dans laquelle ledit deuxième agent tensioactif est présent dans ladite solution aqueuse en une proportion de 5 à 70 parties en poids pour 100 parties en poids dudit diméthylpolysiloxane dans ladite émulsion brute.

**7.** Une composition cosmétique comprenant une microémulsion ayant une dimension particulaire d'émulsion inférieure ou égale à 0,15 micromètre formée par le procédé comprenant :

la formation d'une émulsion brute à partir d'eau, d'une proportion d'un premier agent tensioactif suffisante pour former ladite émulsion brute et de diméthylpolysiloxane de la formule générale :

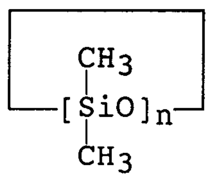 ,

dans laquelle n est un entier ayant une valeur de 3 à 10 ; l'addition graduelle de ladite émulsion brute à une solution aqueuse contenant une quantité catalytique d'un catalyseur de polymérisation en émulsion et un deuxième agent tensioactif ; la polymérisation en émulsion dans un domaine de températures de 0 à 90°C pour atteindre une viscosité prédéterminée ; puis la neutralisation dudit catalyseur de polymérisation,

dans laquelle ledit premier agent tensioactif est choisi dans le groupe (A) formé par les agents tensioactifs cationiques, anioniques et non ioniques, et ledit premier agent tensioactif est un agent tensioactif unique ou une combinaison d'agents tensioactifs choisis dans ledit groupe (A), avec l'exception qu'un agent tensioactif cationique ne peut être combiné avec un agent tensioactif anionique, ledit deuxième agent tensioactif est choisi dans le groupe (B) formé par les agents tensioactifs cationiques, anioniques et non ioniques et ledit deuxième agent tensioactif est un agent tensioactif unique ou une combinaison d'agents tensioactifs choisis dans ledit groupe (B), avec l'exception qu'un agent tensioactif cationique ne peut être combiné avec un agent tensioactif anionique ; et ledit catalyseur de polymérisation est choisi dans le groupe formé par les catalyseurs de polymérisation cationiques et anioniques et les hydroxydes d'ammonium quaternaire, avec les conditions :

lorsque ledit premier agent tensioactif est anionique, ledit catalyseur de polymérisation doit être anionique et ledit deuxième agent tensioactif est choisi parmi les agents tensioactifs anioniques et non ioniques dudit groupe (B) ;

lorsque ledit premier agent tensioactif est cationique, ledit catalyseur de polymérisation doit être cationique et ledit deuxième agent tensioactif est choisi parmi les agents tensioactifs cationiques et non ioniques dudit groupe (B);

lorsque ledit premier agent tensioactif est non ionique et que ledit catalyseur de polymérisation est anionique, ledit deuxième agent tensioactif est choisi parmi les agents tensioactifs anioniques et non ioniques dudit groupe (B) ;

lorsque ledit premier agent tensioactif est non ionique et que ledit catalyseur de polymérisation est cationique, ledit deuxième agent tensioactif est choisi parmi les agents tensioactifs cationiques et non ioniques dudit groupe (B),

et de 0 à 99,9 %, par rapport au poids total de la composition cosmétique, de constituants cosmétiques librement choisis dans le groupe formé par les hydrocarbures, les esters, les alcools supérieurs, les acides gras supérieurs, les rétenteurs d'humidité, les poudres minérales, les poudres organiques, les solvants tels que l'éthanol, les agents tensioactifs, l'eau, les germicides et les parfums.

**8.** Une composition selon la revendication 7, dans laquelle lesdits hydrocarbures sont choisis dans le groupe formé par les paraffines liquides, la vaseline, les paraffines solides et le squalane,

lesdits oligomères oléfiniques sont choisis dans le groupe formé par les esters palmitate d'isopropyle, stéarate de stéaryle, myristate d'octyldodécyle, oléate d'octyldodécyle et triglycéride de l'acide 2-éthylhexanoïque,

lesdits alcools supérieurs sont choisis dans le groupe formé par l'alcool laurylique, l'alcool cétylique et l'alcool stéarylique,

lesdits acides gras supérieurs sont choisis dans le groupe formé par l'acide palmitique et l'acide stéarique,

lesdits rétenteurs d'humidité sont choisis dans le groupe formé par l'éthylène glycol, le propylène glycol, le 1,3-butylène glycol, le glycérol et le sorbitol,

lesdites poudres minérales sont choisies dans le groupe formé par l'oxyde de titane, le noir de carbone, l'oxyde de fer, la séricite, le talc, le kaolin et le mica,

lesdites poudres organiques sont choisies dans le groupe formé par le nylon, le polystyrène, le polyéthylène et les polyacrylates, et

lesdits catalyseurs/agents tensioactifs sont choisis dans le groupe formé par l'acide chlorhydrique, l'acide sulfurique, l'acide phosphorique, l'hydroxyde de potassium, l'hydroxyde de sodium, l'hydroxyde de tétraméthylammonium, l'hydroxyde d'octyltriméthylammonium, l'hydroxyde de dodécyltriméthylammonium, l'hydroxyde d'hexadécyltriméthylammonium, l'hydroxyde d'octyldiméthylbenzylammonium, l'hydroxyde de décyldiméthylbenzylammonium, l'hydroxyde de didodécyldiméthylammonium, l'hydroxyde de dioctadécyldiméthylammonium, l'hydroxyde de suif triméthylammonium et l'hydroxyde de cocotriméthylammonium.

## Patentansprüche

1. Kosmetische Zusammensetzung, enthaltend eine Dimethylpolysiloxanmicroemulsion, wobei diese Microemulsion gebildet wird durch Emulsionspolymerisation von Dimethylpolysiloxan, wobei die durchschnittliche Teilchengröße der Microemulsion $\leqq 0{,}15\ \mu m$ ist.

2. Zusammensetzung, enthaltend eine Dimethylpolysiloxanmicroemulsion, worin die Microemulsion gebildet wird durch Emulsionspolymerisation von Dimethylpolysiloxan der allgemeinen Formel:

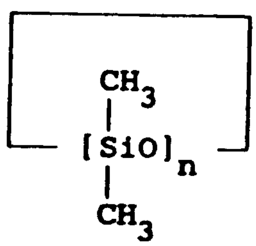

,

worin n eine ganze Zahl mit einem Wert von 3 bis 10 ist und wobei die durchschnittliche Teilchengröße der Microemulsion $\leqq 0{,}15\ \mu m$ ist und der Polymerisationsgrad des polymerisierten Dimethylpolysiloxans der Microemulsion 3 bis 5000 ist.

3. Zusammensetzung mit einer Emulsionsteilchengröße von $\leqq 0{,}15\ \mu m$, gebildet durch ein Verfahren, umfassend: daß man eine Rohemulsion aus Wasser, einer solchen Menge eines ersten oberflächenaktiven Mittels (A), die ausreicht, um diese Rohemulsion zu bilden, und einem Dimethylpolysiloxan der allgemeinen Formel:

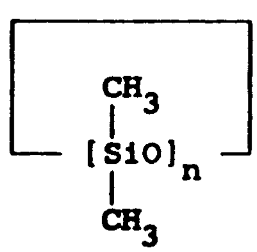

,

worin n eine ganze Zahl mit einem Wert von 3 bis 10 ist, bildet; nach und nach die Rohemulsion zu einer wäßrigen Lösung, die eine katalytische Menge eines die Emulsionspolymerisation fördernden Katalysators und ein zweites oberflächenaktives Mittel (B) enthält, zugibt; die Emulsionspolymerisation bei einer Temperatur im Bereich von 0 bis 90 °C ausführt, um eine vorbestimmte Viskosität zu erreichen und dann den Polymerisationskatalysator neutralisiert.

**4.** Zusammensetzung nach Anspruch 3, worin die anionischen oberflächenaktiven Mittel der Gruppen (A) und (B) ausgewählt sind aus der Gruppe bestehend aus Alkylsulfaten, Alkylbenzolsulfonsäuren, den Sulfatestern von Monoalkylpolyoxyethylenethern und Alkylnaphthylsulfonsäuren, die kationischen oberflächenaktiven Mittel der Gruppen (A) und (B) ausgewählt sind aus der Gruppe bestehend aus quaternären Ammoniumhydroxiden und den entsprechenden Salzen dieser Materialien und die nichtionischen oberflächenaktiven Mittel der Gruppen (A) und (B) ausgewählt sind aus der Gruppe bestehend aus Polyoxyalkylenalkylethern, Polyoxyalkylenalkylphenolethern, Polyoxyalkylenalkylestern, Polyoxyalkylensorbitanalkylestern, Polyethylenglycolen, Polypropylenglycolen und Diethylenglycolen.

**5.** Zusammensetzung nach Anspruch 3, worin das erste oberflächenaktive Mittel in der Rohemulsion in einer Menge von 2 bis 10 Gewichtsprozent enthalten ist und Wasser in der Rohemulsion in einer Menge von 10 bis 40 Gewichtsprozent enthalten ist, wobei der Ausgleich der Rohemulsion Dimethylpolysiloxan ist.

**6.** Zusammensetzung nach Anspruch 3, worin das zweite oberflächenaktive Mittel in der wäßrigen Lösung in einer Menge von 5 bis 70 Gewichtsteilen pro 100 Gewichtsteilen Dimethylpolysiloxan in der Rohemulsion enthalten ist.

**7.** Kosmetische Zusammensetzung, enthaltend eine Microemulsion mit einer Emulsionsteilchengröße von ≦ 0,15 µm, gebildet durch das Verfahren, umfassend: daß man eine Rohemulsion aus Wasser, einer Menge eines ersten oberflächenaktiven Mittels, die ausreicht, um die Rohemulsion zu bilden, und Dimethylpolysiloxan der allgemeinen Formel:

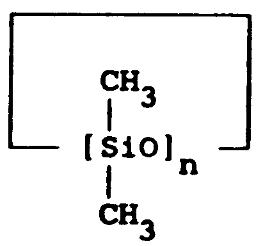

,

worin n eine ganze Zahl mit einem Wert von 3 bis 10 ist, herstellt; nach und nach die Rohemulsion zu einer wäßrigen Lösung, die eine katalytische Menge eines die Emulsionspolymerisation fördernden Katalysators und ein zweites oberflächenaktives Mittel enthält, zugibt; die Emulsionspolymerisation bei einer Temperatur im Bereich von 0 bis 90 ° C durchführt, bis eine vorbestimmte Viskosität erreicht ist; dann den Polymerisationskatalysator neutralisiert; wobei das erste oberflächenaktive Mittel ausgewählt ist aus der Gruppe (A), bestehend aus kationischen, anionischen und nichtionischen oberflächenaktiven Mitteln, und das erste oberflächenaktive Mittel ein einzelnes oberflächenaktives Mittel oder eine Kombination von oberflächenaktiven Mitteln, ausgewählt aus der Gruppe (A), ist, mit der Ausnahme, daß ein kationisches oberflächenaktives Mittel nicht mit einem anionischen oberflächenaktiven Mittel kombiniert werden kann, das zweite oberflächenaktive Mittel ausgewählt ist aus der Gruppe (B), bestehend aus kationischen, anionischen und nichtionischen oberflächenaktiven Mitteln, und das zweite oberflächenaktive Mittel ein einziges oberflächenaktives Mittel oder eine Kombination von oberflächenaktiven Mitteln, ausgewählt aus der Gruppe (B), ist, mit der Ausnahme, daß ein kationisches oberflächenaktives Mittel nicht mit einem anionischen oberflächenaktiven Mittel kombiniert werden kann, und der Polymerisationskatalysator ausgewählt ist aus der Gruppe bestehend aus kationischen und anionischen Polymerisationskatalysatoren und quaternären Ammoniumhydroxiden mit folgenden Bedingungen: wenn das erste oberflächenaktive Mittel ein anionisches oberflächenaktives Mittel ist, muß der Polymerisationskatalysator anionisch sein, und das zweite oberflächenaktive Mittel wird ausgewählt aus den anionischen und nichtionischen oberflächenaktiven Mitteln der Gruppe (B); wenn das erste oberflächenaktive Mittel kationisch ist, muß der Polymerisationskatalysator kationisch sein, und das zweite oberflächenaktive Mittel wird aus den kationischen und nichtionischen oberflächenaktiven Mitteln der Gruppe (B) ausgewählt; wenn das erste oberflächenaktive Mittel nichtionisch ist und der Polymerisationskatalysator anionisch ist, wird das zweite oberflächenaktive Mittel ausgewählt aus den anionischen und nichtionischen oberflächenaktiven Mitteln der Gruppe (B), und wenn das erste oberflächenaktive Mittel nichtionisch ist und der Polymerisationskatalysator kationisch ist, wird das zweite oberflächenaktive Mittel ausgewählt aus den kationischen und nichtionischen oberflächenaktiven Mitteln der Gruppe (B), und 0 bis 99,9 Gewichtsprozent, bezogen auf die Gesamtmenge der kosmetischen Zusammensetzung frei ausgewählter kosmetischer Komponenten, ausgewählt aus der Gruppe bestehend aus Kohlenwasserstoffen, Estern, höheren Alkoholen, höheren Fettsäuren, Feuchthaltemitteln, anorganischen Pulvern, organischen Pulvern, Lösungsmitteln, wie Ethanol, oberflächenaktiven Mitteln, Wasser, Germiciden und Parfums.

**8.** Kosmetische Zusammensetzung nach Anspruch 7, worin die Kohlenwasserstoffe ausgewählt sind aus der Gruppe bestehend aus flüssigem Paraffin, Vaseline, festen Paraffinen und Squalan, die Olefinoligomere ausgewählt sind aus der Gruppe von Estern bestehend aus Isopropylpalmitat, Stearylstearat, Octyldodecylmyristat, Octyldodecyloleat und 2-Ethylhexansäuretriglycerid, die höheren Alkohole ausgewählt sind aus der Gruppe bestehend aus Laurylalkohol, Cetylalkohol und Stearylalkohol, die höheren Fettsäuren ausgewählt sind aus der Gruppe bestehend aus Palmitinsäure und Stearinsäure, die Feuchthaltemittel ausgewählt sind aus der Gruppe bestehend aus Ethylenglycol, Propylenglycol, 1,3-Butylenglycol, Glycerin und Sorbit, die anorganischen Pulver ausgewählt sind aus der Gruppe bestehend aus Titanoxid, Ruß, Eisenoxid, Sericit, Talkum, Kaolin und Glimmer, die organischen Pulver ausgewählt sind aus der Gruppe bestehend aus Nylon, Polystyrol, Polyethylen und Polyacrylat, und die Katalysatoren/ oberflächenaktiven Mittel ausgewählt sind aus der Gruppe bestehend aus Chlorwasserstoffsäure, Schwefelsäure, Phosphorsäure, Kaliumhydroxid, Natriumhydroxid, Tetramethylammoniumhydroxid, Octyltrimethylammoniumhydroxid, Dodecyltrimethylammoniumhydroxid, Hexadecyltrimethylammoniumhydroxid, Octyldimethylbenzylammoniumhydroxid, Decyldimethylbenzylammoniumhydroxid, Didodecyldimethylammoniumhydroxid, Dioctadecyldimethylammoniumhydroxid, talkartigem Trimethylammoniumhydroxid und Cocotrimethylammoniumhydroxid.